(19) Europäisches Patentamt
European Patent Office
Office européen des brevets

(11) **EP 4 375 296 A1**

(12) **EUROPEAN PATENT APPLICATION**

(43) Date of publication:
**29.05.2024 Bulletin 2024/22**

(21) Application number: **23209441.7**

(22) Date of filing: **13.11.2023**

(51) International Patent Classification (IPC):
**C07K 16/18** *(2006.01)* **A61P 25/00** *(2006.01)*
**A61K 39/00** *(2006.01)*

(52) Cooperative Patent Classification (CPC):
**C07K 16/18; A61P 25/00;** A61K 2039/505;
A61P 3/06; A61P 9/00; C07K 2317/24;
C07K 2317/34; C07K 2317/56; C07K 2317/565;
C07K 2317/76; C07K 2317/92

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.11.2022 CN 202211439397**

(71) Applicants:
• **Versitech Limited
Pokfulam, Hong Kong (HK)**
• **Shenzhen Institute of Advanced Technology Chinese Academy of Sciences
Nanshan Shenzhen (CN)**

(72) Inventors:
• **HOO, Lai Chong Ruby
Pokfulam (HK)**

• **XU, Aimin
Pokfulam (HK)**
• **LIAO, Boya
Pokfulam (HK)**
• **CHANG, Junlei
Shenzhen (CN)**
• **YANG, Shilun
Shenzhen (CN)**
• **LI, Simeng
Shenzhen (CN)**

(74) Representative: **HGF
HGF Limited
1 City Walk
Leeds LS11 9DX (GB)**

Remarks:
The complete document including Reference Table(s) and the Sequence Listing(s) can be downloaded from the EPO website

(54) **AN A-FABP NEUTRALIZING MONOCLONAL ANTIBODY AND PREPARATION METHOD AND USE THEREOF**

(57) The present invention provides an A-FABP neutralizing monoclonal antibody or antigen-binding fragment thereof and a preparation method and use thereof, which comprises: complementarity determining regions 1 to 3 of a heavy chain variable region, the amino acid sequences of which are set forth in SEQ ID NOs. 1-3, respectively, and/or complementarity determining regions 1-3 of the light chain variable region, the amino acid sequences of which are set forth in SEQ ID NOs. 4-6. This monoclonal antibody is highly specific for A-FABP and has no cross-reactivity with human and mouse epidermal FABP and heart FABP; it has a good binding affinity for human A-FABP and mouse A-FABP; and its treatment alleviates MCAO-induced ischemic stroke injury in mice, which is associated with alleviated BBB disruption and reduced activation of pro-inflammatory JNK signaling; treatment of healthy mice for 21 days does not change physiological parameters, indicating its safety.

Fig. 1

# Description

## Technical Field

The invention belongs to the fields of immunology and biopharmaceuticals, and specifically relates to a monoclonal antibody capable of neutralizing free adipocyte fatty acid-binding protein in blood circulation, its preparation method, and evaluation of its use in the treatment of ischemic stroke.

## Background of the Invention

Ischemic stroke (IS) is the second leading cause of death worldwide and the third leading combined cause of death and disability. Currently, the only drug approved by the U.S. Food and Drug Administration for the treatment of ischemic stroke is tissue plasminogen activator (tPA). However, the use of tPA is limited by its narrow therapeutic window (within 4.5 hours after stroke onset) and severe side effects, including cerebral hemorrhage and angioedema. Therefore, only a minority of patients benefit from such thrombolytic therapy. For patients who are not eligible for t-PA therapy, mechanical thrombectomy (mTE) can be used to remove the thrombus, but this is only feasible for patients with large artery occlusion. Furthermore, the risks of mechanical thrombectomy include intracerebral hemorrhage, vascular injury, vasospasm, and stent-related complications, which outweigh its benefits. Therefore, it is of great significance to develop a novel and effective therapy for ischemic stroke.

Adipocyte fatty acid-binding protein (A-FABP) is also known as A-FABP or AP2. It is an adipokine that is abundantly expressed in adipocytes and macrophages and can be secreted into the circulation. In addition to its function as a fatty acid chaperone, A-FABP is also a key mediator of inflammation and plays a deleterious role in mediating cerebral ischemic stroke[2]. High circulating level of A-FABP has been found in patients with metabolic complications, including obesity, arteriosclerosis, and type 2 diabetes. In mice, gene deletion or pharmacological inhibition of A-FABP can ameliorate the adverse effects of metabolic disorders such as obesity, lipid metabolism, liver cirrhosis, and glucose metabolism.

The level of circulating A-FABP is also closely related to increased infarct size and early death, and is an independent prognostic biomarker for patients with acute ischemic stroke. Previous study by the inventors showed that circulating and cerebral A-FABPs were elevated in mice after ischemic stroke injury. Peripheral blood monocytes, microglia, and infiltrating monocytes are the main cellular sources of circulating and cerebral A-FABPs, respectively. A-FABP exacerbates ischemic stroke injury by regulating matrix metallopeptidase 9 (MMP-9)-mediated blood-brain barrier (BBB) disruption. Gene deletion of A-FABP or pharmacological inhibition of A-FABP using its selective inhibitor BMS309403 significantly reduces the prognosis of ischemic stroke and reduces BBB disruption and post-stroke brain inflammation. These studies suggest that A-FABP is a potential therapeutic target for ischemic stroke injury.

Although blocking the activity of A-FABP with BMS309403 was shown to be effective in alleviating type 2 diabetes and atherosclerosis, steatohepatitis, liver fibrosis, and ischemic stroke in mouse models, BMS309403 is a small molecule drug with low specificity and off-target activities, such as inhibition of cardiomyocyte contractility and stimulation of glucose uptake in C2C12 myotubes. These properties limit the development of its clinical applications.

There is an increasing trend of using antibody drugs for treatment of various diseases owing to their higher specificity, fewer off-target side effects, fewer drug-drug interaction and relatively long half-life A recent study also showed that a monoclonal antibody against secreted A-FABP exhibits antidiabetic effects in obese mice. Therefore, monoclonal antibodies targeting circulating A-FABP may be a potential treatment for ischemic stroke.

Gökhan's research group invented and revealed the therapeutic effect of a monoclonal antibody named CA33 targeting serum A-FABP[3]. It is shown that treatment with CA33 significantly improves the glucose metabolism in obese mouse models. The study by Cao in 2013 showed that neutralization of A-FABP with polyclonal antibodies reverses obesity-induced diabetes, indicating that A-FABP is a potential target for the treatment of diabetes[4].

Most of the developed monoclonal antibody drugs target the molecules in the signaling pathways that regulate post-stroke inflammation, neuronal repair and regeneration. As the inflammatory response in humans and rodents upon ischemia insult are different, most monoclonal antibodies that have shown beneficial effects in animal studies have failed in clinical trials. There is currently a lack of effective antibody drugs for the treatment of ischemic stroke injury.

## Summary of the Invention

Therefore, the purpose of the present invention is to use traditional hybridoma technology to generate monoclonal antibodies capable of neutralizing circulating A-FABP.

The inventors of the present invention found through research that the level of A-FABP in the blood circulation of mice after middle cerebral artery occlusion (MCAO) surgery was significantly increased; and that the ischemic stroke outcome is significantly alleviated in A-FABP-deficient mice subjected to MCAO surgery, compared to that of wild-type mice. Replenishment of recombinant A-FABP in the circulation of A-FABP-deficient mice worsens the ischemic stroke outcome. These results indicate that the deleterious effects of circulating A-FABP in mice with ischemic stroke are consistent with those in humans.

In an *in vitro* screening study, the inventor of the present invention found that a monoclonal antibody (mAb) named "6H2" has a strong neutralizing ability against adipocyte fatty acid-binding protein (A-FABP), and the study showed as follows:

(1) 6H2 is highly specific for A-FABP and has no cross-reactivity with human and mouse epidermal FABP (E-FABP) and heart FABP (H-FABP).

(2) 6H2 has good binding affinity to human A-FABP and mouse A-FABP.

(3) Treatment with 6H2 alleviates the blood-brain barrier disruption and attenuates the activation of pro-inflammatory JNK signaling caused by MCAO, thereby alleviating ischemic stroke injury in mice.

(4) Mechanistically, 6H2 may interfere the interaction between free fatty acid (FFA) and A-FABP by interacting with the lid of the fatty acid (FFA) binding pocket of A-FABP. Treatment with 6H2 alleviates the harmful effects of A-FABP in ischemic stroke.

(5) Injection of healthy mice with 6H2 for 21 days does not alter their physiological parameters, indicating the safety of 6H2.

Based on these results, the first aspect of the present invention provides an A-FABP neutralizing monoclonal antibody or antigen-binding fragment thereof, comprising:

a complementarity determining region 1 of a heavy chain variable region, the amino acid sequence of which is set forth in SEQ ID No. 1; a complementarity determining region 2 of the heavy chain variable region, the amino acid sequence of which is set forth in SEQ ID No. 2; and a complementarity determining region 3 of the heavy chain variable region, the amino acid sequence of which is set forth in SEQ ID No. 3, and/or

a complementarity determining region 1 of a light chain variable region, the amino acid sequence of which is set forth in SEQ ID No. 4; a complementarity determining region 2 of the light chain variable region, the amino acid sequence of which is set forth in SEQ ID No. 5; and a complementarity determining region 3 of the light chain variable region, the amino acid sequence of which is set forth in SEQ ID No. 6.

The amino acid sequence of CDR1 of the heavy chain variable region is: DYNMD (SEQ ID NO. 1).

The amino acid sequence of CDR2 of the heavy chain variable region is: DINPYNGDTIYNQNFKG (SEQ ID NO. 2).

The amino acid sequence of CDR3 of the heavy chain variable region is: GYGF (SEQ ID NO. 3).

The amino acid sequence of CDR1 of the light chain variable region is: RSSQSLVYINGNTYLH (SEQ ID NO. 4).

The amino acid sequence of CDR2 of the light chain variable region is: KVSNRFS (SEQ ID NO. 5).

The amino acid sequence of CDR3 of the light chain variable region is: SQSTHVPYT (SEQ ID NO. 6).

In the A-FABP neutralizing monoclonal antibody or antigen-binding fragment thereof of the present invention, VHCDR1, VHCDR2 and VHCDR3, the amino acid sequences of which are set forth in SEQ ID Nos. 1-3, respectively, and VLCDR1, VLCDR2 and VLCDR3, the amino acid sequences of which are set forth in SEQ ID Nos. 4-6, respectively, are complementarity determining regions (CDRs) defined according to Kabat.

As a preferred embodiment of the present invention, the amino acid sequence of the heavy chain variable region of the A-FABP neutralizing monoclonal antibody is set forth in SEQ ID NO. 7, and the amino acid sequence of the light chain variable region is set forth in SEQ ID NO. 8.

The amino acid sequence of the heavy chain variable region (SEQ ID NO. 7) is:

VKLQESGPELVKPGASVKIPCKASGYTFTDYNMDWVRQSRGKSLE

WIGDINPYNGDTIYNQNFKGKATLTVDKSSTTAYLELRSLTSEDTAVYYC

ASGYGFWGQGTTVTVSS.

The amino acid sequence of the light chain variable region (SEQ ID NO. 8) is:

DIVMTQAPLSLPVSLGAQASISCRSSQSLVYINGNTYLHWYLQKPGQ SPKLLIYKVSNRFSGVPDRFSGSGSGTDFTLKISRVEAEDLGVYFCSQSTH VPYTFGGGTKLEIK.

As an embodiment of the present invention, the antigen-binding fragment of the A-FABP neutralizing monoclonal antibody is selected from one or more of Fab, Fab', (Fab')2, Fv, disulfide-linked Fv, scFv, diabody, or single-domain antibody (sdAb).

As an embodiment of the present invention, the A-FABP neutralizing monoclonal antibody is selected from one or more of a murine antibody, a chimeric antibody, a humanized antibody, a bispecific antibody, or a multispecific antibody.

As a preferred embodiment of the present invention, the A-FABP neutralizing monoclonal antibody is a murine antibody.

Preferably, the amino acid sequence of the heavy chain variable region of the murine antibody is set forth in SEQ ID NO. 7, and the amino acid sequence of the light chain variable region is set forth in SEQ ID NO. 8.

Alternatively, the A-FABP neutralizing monoclonal antibody is a humanized antibody. Preferably, the humanized antibody comprises human IgG1, IgG2, IgG3 or IgG4; more preferably, the humanized antibody comprises human IgG4.

Preferably, the amino acid sequence of the heavy chain of the humanized antibody is set forth in SEQ ID NO. 9, and the amino acid sequence of the light chain is set forth in SEQ ID NO. 10.

The amino acid sequence of the heavy chain of humanized 6H2 antibody is:

VKLQESGPELVKPGASVKIPCKASGYTFTDYNMDWVRQSRGKSLEW IGDINPYNGDTIYNQNFKGKATLTVDKSSTTAYLELRSLTSEDTAVYYCAS GYGFWGQGTTVTVSSASTKGPSVFPLAPCSRSTSESTAALGCLVKDYFPE PVTVSWNSGALTSGVHTFPAVLQSSGLYSLSSVVTVPSSSLGTKTYTCNVD HKPSNTKVDKRVESKYGPPCPSCPAPEFLGGPSVFLFPPKPKDTLMISRTPE VTCVVVDVSQEDPEVQFNWYVDGVEVHNAKTKPREEQFNSTYRVVSVL TVLHQDWLNGKEYKCKVSNKGLPSSIEKTISKAKPSVFLFPPKPKDTLMIS RTPEVTCVVVDVSQEDPEVQFNWYVDGVEVHNAKTKPREEQFNSTYRV VSVLTVLHQDWLNGKEYKCKVSNKGLPSSIEKTISGQPREPQVYTLPPSQ EEMTKNQVSLTCLVKGFYPSDIAVEWESNGQPENNYKTTPPVLDSDGSFF LYSRLTVDKSRWQEGNVFSCSVMHEALHNHYTQKSLSLSLGKPQVYTLP PSQEEMTKNQVSLTCLVKGFYPSDIAVEWESNGQPENNYKTTPPVLDSDG SFFLYSRLTVDKSRWQEGNVFSCSVMHEALHNHYTQKSLS (SEQ ID NO.9).

The amino acid sequence of the light chain of humanized 6H2 antibody is:

DIVMTQAPLSLPVSLGAQASISCRSSQSLVYINGNTYLHWYLQKPGQ

SPKLLIYKVSNRFSGVPDRFSGSGSGTDFTLKISRVEAEDLGVYFCSQSTH VPYTFGGGTKLEIKPTVTLFPPSSEELQANKATLVCLISDFYPGAVTVAWK ADGSPVKAGVETTKPSKQSNNKYAASSYLSLTPEQWKSHRSYSCQVTHE GSTVEKTVA (SEQ ID NO.10).

The second aspect of the present invention provides a preparation method for the A-FABP neutralizing monoclonal antibody or antigen-binding fragment thereof, comprising:

(1) immunizing a mouse with a human recombinant A-FABP peptide;
(2) preparing hybridoma cells by fusing spleen cells from the immunized mouse in step (1) with myeloma cells;
(3) screening hybridoma cells which produce the A-FABP neutralizing monoclonal antibody.

As an embodiment of the present invention, the immunizing is performed after mixing the human recombinant A-FABP peptide in step (1) with an adjuvant (AbMax Biotechnology Co., Ltd. (Beijing), product number AD11.15).

As an embodiment of the present invention, the screening in step (3) comprises: culturing with a hypoxanthine-aminopterin-thymidine medium, followed by screening hybridoma cells which produce the A-FABP neutralizing monoclonal antibody by enzyme-linked immunosorbent assay;

As an embodiment of the present invention, the preparation method may further comprise:
(4) preparing the A-FABP neutralizing monoclonal antibody from mouse ascites and purifying with protein G magnetic beads. Wherein, the protein G magnetic beads are, for example, Chinese GenScript, L00209.

The third aspect of the present invention provides a pharmaceutical composition for preventing and/or treating elevated circulating A-FABP or a disease or symptom resulting therefrom, comprising the A-FABP neutralizing monoclonal antibody or antigen-binding fragment thereof of the present invention, or an A-FABP neutralizing monoclonal antibody or antigen-binding fragment thereof prepared according to the preparation method of the present invention.

As an embodiment of the present invention, the pharmaceutical composition further comprises a pharmaceutically acceptable carrier, diluent, and/or adjuvant.

As an embodiment of the present invention, the pharmaceutical composition further comprises one or more other pharmaceutical active ingredients for preventing and/or treating elevated circulating A-FABP or a disease or symptom resulting therefrom.

Wherein, the elevated circulating A-FABP or the disease or symptom resulting therefrom may be selected from one or more of ischemic stroke, obesity, liver cirrhosis, arteriosclerosis, and diabetes.

The fourth aspect of the present invention provides the use of the A-FABP neutralizing monoclonal antibody or antigen-binding fragment thereof of the present invention, or the A-FABP neutralizing monoclonal antibody or antigen-binding fragment thereof prepared according to the preparation method of the present invention, in the manufacture of a medicament for preventing and/or treating elevated circulating A-FABP or a disease or symptom resulting therefrom.

As an embodiment of the present invention, the medicament further comprises a pharmaceutically acceptable carrier, diluent, and/or adjuvant.

As an embodiment of the present invention, the medicament further comprises one or more other pharmaceutical active ingredients for preventing and/or treating elevated circulating A-FABP or the disease or symptom resulting therefrom.

Accordingly, the fifth aspect of the present invention provides a method for preventing and/or treating elevated circulating A-FABP or a disease or symptom resulting therefrom, comprising administering the A-FABP neutralizing monoclonal antibody or antigen-binding fragment thereof of the present invention, or the A-FABP neutralizing monoclonal antibody or antigen-binding fragment thereof prepared according to the preparation method of the present invention to a subject in need thereof.

As an embodiment of the present invention, the method further comprises administering one or more other pharmaceutical active ingredients for preventing and/or treating elevated circulating A-FABP or the disease or symptom resulting therefrom, simultaneously or at intervals with administering the A-FABP neutralizing monoclonal antibody or antigen-binding fragment thereof of the present invention, or the A-FABP neutralizing monoclonal antibody or antigen-binding fragment thereof prepared according to the preparation method of the present invention to a subject in need thereof.

As a specific embodiment of the present invention, the disease or symptom resulting from the elevated circulating A-FABP is selected from one or more of ischemic stroke, obesity, liver cirrhosis, arteriosclerosis, and diabetes.

The A-FABP neutralizing monoclonal antibody provided by the present invention is highly specific for A-FABP and has no cross-reactivity with human and mouse epidermal FABP and heart FABP; it has a good binding affinity for human A-FABP and mouse A-FABP; and its treatment alleviates MCAO-induced ischemic stroke injury in mice, which is associated with alleviated BBB disruption and reduced activation of pro-inflammatory JNK signaling; injection into healthy mice for 21 days does not change physiological parameters, indicating its safety.

**Description of the Drawings**

In the following, the embodiments of the present invention are described in detail with reference to the accompanying drawings, wherein:

Fig. 1 shows representative photographs of coronal brain sections stained with TTC in A-FABP KO mice 24 hours after MCAO or sham surgery after infusion of vehicle or recombinant A-FABP protein (rA-FABP);
Fig. 2 shows comparative plots of the relative infarct volume, brain water content, neurological score (n=5), and survival rate (n=9) in A-FABP KO mice 24 hours or 7 days after MCAO or sham surgery after infusion of vehicle or rA-FABP;
Fig. 3 shows a structural analysis diagram of molecular docking of the A-FABP-6H2 complex;
Fig. 4 shows the ELISA assay results of the antigen-binding specificity of 6H2 with recombinant mouse or human A-FABP, E-FABP, and H-FABP;
Fig. 5 shows the neutralizing activity of 6H2 on rA-FABP-induced JNK activation;
Fig. 6 shows the neutralizing effect of 6H2 on rA-FABP-induced MMP-9 production;
Fig. 7 shows the results of half-life of 6H2 in serum;
Fig. 8 shows representative photographs of TTC-stained coronal brain sections and a comparative plot of the relative infarct volume in mice treated with vehicle or 6H2 (1.8 mg/kg or 3.6 mg/kg) at 24 hours after MCAO surgery;
Fig. 9 shows the dynamic change of circulating A-FABP levels (A), brain water content percentage (B), neurological score (C), and survival rate (D) in mice treated with vehicle or 6H2 after MCAO or sham surgery;
Fig. 10 shows the relative mRNA abundance of inflammatory cytokines in the brains of mice treated with vehicle or 6H2 (1.8 mg/kg) after MCAO or sham surgery.
Fig. 11 shows representative photographs of mouse brains stained with Evans blue 24 hours after MCAO or sham surgery and a comparative plot of Evans blue concentration (n=5);
Fig. 12 shows representative immunoblots of tight junction (TJ) proteins and the band intensity of each protein relative to GAPDH, in the brains of mice treated with vehicle or 6H2 after MCAO or sham surgery;
Fig. 13 shows representative immunoblots of A-FABP, MMP-9, p-JNK (Thr183/Tyr185), and p-c-Jun (Ser 63/73), and the band intensity of each protein relative to its respective control protein or GAPDH, in the cerebral cortex of mice treated with vehicle or 6H2 after MCAO or sham surgery (n=5);
Fig. 14 shows the levels of circulating MMP-9 in the mice treated with vehicle or 6H2 after MCAO or sham surgery (n=10);
Fig. 15 shows a schematic diagram of monitoring the metabolic profile of healthy mice injected with 6H2 or vehicle for 21 days;
Fig. 16 shows the changes in body weight (A); changes in fat mass (B) and muscle percentage (C) of healthy mice receiving 6H2 or vehicle injection for 21 days; fasting blood glucose in mice 10 days after 6H2 or vehicle injection (D); glucose tolerance test (GTT) in mice 15 days after 6H2 injection (E); and changes in TC, TG and FFA levels in mice during 21-day treatment with 6H2 or vehicle (F-H).

**Detailed Description of the Invention**

The present invention will be further described in detail below with reference to specific embodiments. The examples given are only for illustrating the present invention and are not intended to limit the scope of the present invention.
The experimental methods used in the following examples are all conventional methods unless otherwise specified. The reagents, cell lines, virus strains, plasmids, kits, etc. used in the following examples are all commercially available products unless otherwise specified.
The experimental methods used in the examples are described below.

(1) Determination of the therapeutic potential and safety of 6H2 for ischemic stroke

To evaluate the therapeutic efficiency of the A-FABP neutralizing antibody 6H2 in relieving stroke outcome, mice were intravenously injected with 6H2 (1.8 mg/kg or 3.6 mg/kg) or non-immunoglobulin G (IgG; 1.8 mg/kg) as control one hour after receiving MCAO or sham surgery, followed by injection every 3 days. To determine the safety of 6H2 treatment, healthy C57BL/6N mice were intravenously injected with control IgG or 6H2 (1.8 mg/kg) every 3 days for 21 days. Then various physiological parameters were measured. The experimental protocol was reviewed and approved by the Ethics Committee of the University of Hong Kong (4847-18).

(2) Neurological score assessment and cerebral infarct volume measurement

After mice underwent MCAO or sham surgery and were reperfused for 24 hours, the severity of neurological deficits was assessed according to the following criteria: 0 = no deficit; 1 = failure to extend forepaw; 2 = spontaneously turning to contralateral side; 3 = spontaneously circling to contralateral side; 4 = loss of ability to walk; 5 = death. The mouse brains are then removed and cut into coronal sections.

The cerebral infarct volume was assessed by 2,3,5-triphenyltetrazolium chloride (TTC) staining as described in the inventors' article published in the European Heart Journal[1]. The brain sections were stained in TTC staining solution at 37°C for 15 minutes in the dark, with the red area being normal brain tissue, and the white area being the infarction area. Infarct volume is quantified using Image J software (Image J 1.42 software, National Institutes of Health, USA). The calculation formula is as follows:

$$[\text{contralateral hemisphere (mm}^3) - \text{undamaged ipsilateral hemisphere (mm}^3)]/\text{area of the contralateral hemisphere} \times 100\%.$$

(3) Corner test and survival rate

In the corner test, two wooden boards (30 cm*20 cm* 1 cm) were connected at an angle of 30° to form a corner. The mouse was placed midway from the corner. When the mice reach the corner, they crawl upwards and then turn to either side. The number of left turns (ipsilateral side) during the tests was recorded.

In survival rate experiments, the number of living animals in each group was recorded and its percentage was calculated.

(4) Brain water content

The brains of mice were collected after reperfusion 24 hours after MCAO surgery. Wet tissue samples were weighed immediately after euthanasia. Brain samples were dried in an oven at 110°C for 48 hours. The samples were weighed before and after the drying process. The formula for calculating brain water content is as follows:

$$[(\text{wet tissue weight-dry tissue weight})/\text{wet tissue weight}] \times 100\%.$$

(5) Western blot analysis

Proteins were extracted from mouse primary peritoneal macrophages. Cell lysates were separated using sodium dodecyl sulfate (SDS)-polyacrylamide gel electrophoresis. Proteins were transferred to polyvinylidene difluoride membrane and detected with primary antibodies against MMP-9 (AB38898, Abeam, USA) and β-actin (Proteintech, 66009-1-Ig, USA). Membranes were incubated with horseradish peroxidase (HRP)-conjugated secondary antibodies. The intensity of protein bands was quantified using NIH Image J software.

(6) Biochemical and immunological analysis

Blood glucose was measured using an Accu-Check blood glucose meter (Roche, Germany). The concentrations of serum triglyceride (TG) and total cholesterol (TC) were quantified using commercial kits (Stanbio Laboratory, USA) according to the manufacturer's manual. Serum FFA concentrations were measured using FFAs, Half Micro Test (Roche, Germany).

(7) Glucose tolerance test (GTT) and insulin tolerance test (ITT)

In the GTT experiment, mice were fasted overnight (19:00 pm-9:00 am) and then intraperitoneally injected with D-glucose (Sigma#G5767, 2 g/kg body weight).

In the ITT experiment, mice were fasted for 6 hours (8:00 am-14:00 pm) and then intraperitoneally injected with insulin (Actrapid® HM, 1U/kg body weight). Blood was collected from the tail vein at different time points specified in each experiment, and glucose levels were measured with an Accu-Check blood glucose meter.

**Examples**

**Example 1**

This Example is used to construct a mouse model of ischemic stroke.

**1. Preparation of 8-week-old male A-FABP KO mice**

The A-FABP KO mice were prepared with reference to the inventors' article[1]. Briefly, A-FABP KO mice were prepared using a gene-targeted knockout approach that utilized vectors to replace the exons of the A-FABP gene with luciferase and neomycin genes. Genotyping analysis was performed using gene-specific primers to identify WT, A-FABP KO, and heterozygous mice.

Eight-week-old male A-FABP KO mice and their wild-type (WT) siblings or C57BL/6N mice were randomly grouped and housed in a regular 12-hour light/dark cycle with a humidity of 60% to 70% and free access to water and food.

**2. Temporary cerebral artery occlusion**

The mice were anesthetized with 2% isoflurane. Body temperature was maintained at $37 \pm 0.5°C$ with a thermostat-controlled heating pad. After the mice were stabilized under anesthesia state, the middle cerebral artery was occluded by introducing a filament with its head wrapped in silicon through the external carotid artery into the internal carotid artery. After 60 minutes, the filament was removed. During surgery, cortical blood transfusion was monitored using laser Doppler flowmetry. A sudden drop in blood flow below 20-30% of the baseline value is considered adequate occlusion. After 1 hour of occlusion, the blood flow supply will be restored.

**3. Determine the adverse effects of circulating A-FABP on ischemic stroke**

8-week-old male A-FABP KO mice were infused with A-FABP recombinant protein (rA-FABP, AIS, HKU) (2 $\mu$g/h) or vehicle (mainly non-immune immunoglobulins) as a control using an osmotic pump for 7 days followed by Sham or MCAO surgery, and then infused for 23 h or 7 days. Various physiological parameters (including body weight, body fat composition, blood lipid, fasting blood glucose, etc.) were analyzed.

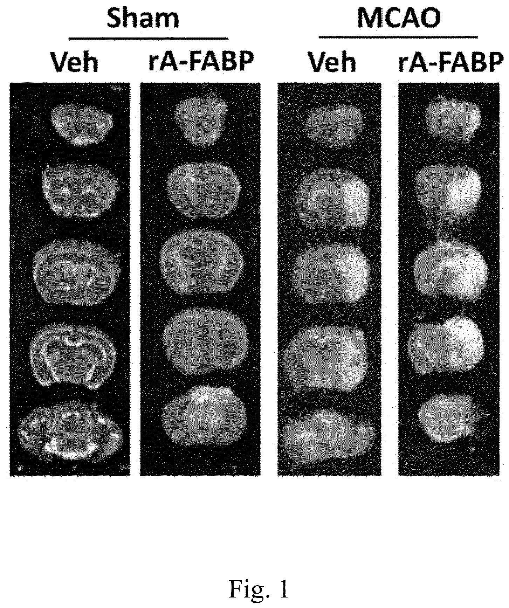

Fig. 1 shows representative photographs of coronal brain sections stained with TTC in A-FABP KO mice with MCAO or sham surgery and reperfusion for 24 hours after infusion of vehicle or recombinant A-FABP protein(n=5).

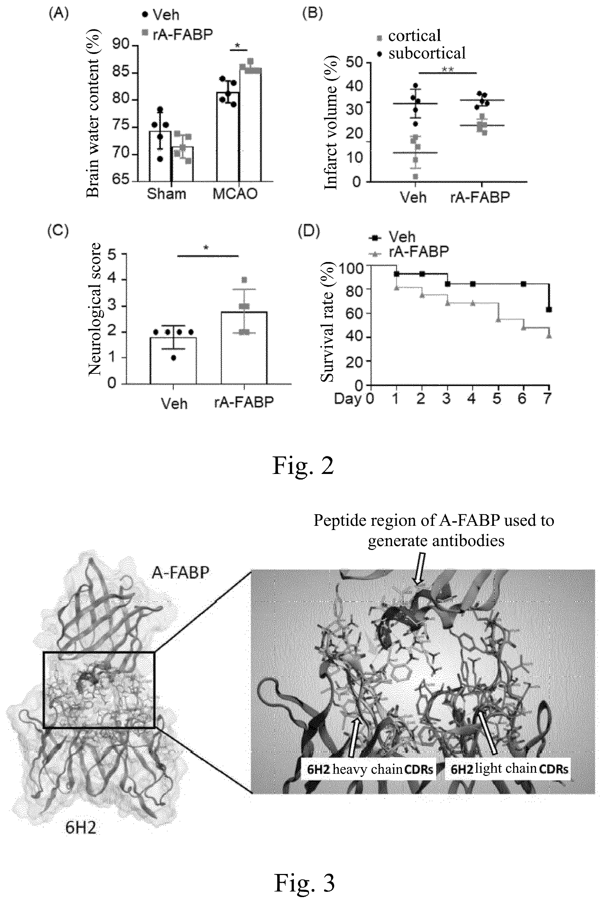

Fig. 2 shows comparative plots of the relative infarct volume, brain water content, neurological score (n=5), and survival rate (n=9) in A-FABP KO mice after MCAO or sham surgery and reperfusion for 24 hours or 7 days after infusion of vehicle or recombinant A-FABP protein; data are represented as mean $\pm$ SD, *P< 0.05, ** P< 0.01.

TTC staining showed that the MCAO surgery-induced infarct volume in A-FABP KO mice infused with recombinant A-FABP protein was significantly increased. Consistently A-FABP KO mice supplemented with recombinant A-FABP protein had significantly increased brain edema, neurological deficits and decreased survival rates. These data confirmed the deleterious role of circulating A-FABP in mediating cerebral ischemic injury.

**Example 2**

The hybridoma technique was used to produce neutralizing monoclonal antibodies against A-FABP. The specific method is as follows:

**1. Antigen**

The peptide sequence from amino acid position 22 to amino acid position 36 of human A-FABP was used as the antigen to immunize mice. The sequence is: KEVGVGFATRKVAGC (SEQ ID No. 11).

1.1 The 70 $\mu$g of antigen was diluted with 500 $\mu$L of sterile water and mixed with 600 $\mu$L of Freund's complete adjuvant. The mixture was sonicated into a milky white mixture using an ultrasonic cell crusher to generate immunogen A.

1.2 The 140 $\mu$g of antigen was diluted with 1 mL sterile water and mixed with 1 mL adjuvant (AbMax Biotechnology Co., Ltd. (Beijing), AD11.15). The mixture was shaken on a shaker at 80-100 rpm at room temperature for 2 hours to generate immunogen B.

**2. Immunization of mice**

Immunogen A was injected intravenously into BALB/c mice every 3 days for 2 weeks.

Immunogen B was given to BALB/c mice via intraperitoneal (i.p.) and intramuscular (i.m.) injection every 3 days for 2 weeks.

**3. Enzyme-linked immunosorbent assay (ELISA)**

Fourteen days after injection, blood samples were collected from the tail veins of immunogen A- or immunogen B-treated mice for ELISA to determine the presence of antibodies against A-FABP.

ELISA plates were coated with 1 μg/ml human or mouse A-FABP recombinant protein (AIS, HKU) overnight at 4°C. 100 μL of serum was added to each well of the ELISA plate and incubated overnight at 4°C. The plates were then washed three times with phosphate-buffered saline (PBS) and blocked with 5% milk in PBS for 1 hour at room temperature. When serum antibody titers reached twice the background value at a dilution of 1:10,000, spleen single cells from immunized mice were fused with myeloma cells SP2/0. The cells were cultured in a xanthine-aminopterin-thymidine selection medium for 10 days before single clone colonies were picked into 96 wells. After 7 days, the antibody concentrations in the supernatants of monoclonal colony were further determined by ELISA.

**4. Preparation of monoclonal antibodies from mouse ascites**

Positive hybridoma cells were injected into the peritoneal cavity of mice for 10 days to produce ascites containing the desired antibodies. The ascitic fluid was collected and centrifuged to remove lipids. Then, according to the manufacturer's manual, the monoclonal antibodies in the ascites were purified with protein G beads (GenScript, L00209, China) to obtain the monoclonal antibody 6H2 of the present invention.

**5. Molecular docking**

In order to evaluate and determine the structure of the A-FABP-6H2 complex and the target epitope, protein-protein docking analysis was performed using Molecular Operating Environment (MOE) software, and the results are shown in Fig. 3.

The crystal structure of A-FABP was retrieved from the Protein Data Bank (PDB, code: 2NNQ). SAbPred (http://opig.stats.ox.ac.uk/webapps/newsabdab/sabpred/) was used to predict the structure of 6H2.

Molecular docking study was performed using the Molecular Operating Environment (MOE) software 2014.9. For the preparation of the proteins, water and co-crystal ligands were removed, hydrogen was added, and other important parameters were adjusted via the MMFF94x force field. The A-FABP protein and 6H2 were then further processed with the prepared protein module to simulate the missing loop region and calculate the ionization of the protein and protonated protein structure. The prepared 6H2 is defined as a receptor, and its binding site is restricted to complementarity determining regions (CDRs). The prepared A-FABP will attack the CDRs of 6H2, after 100 trials, until the most stable docking complex is reached.

The prediction of the target epitope in Fig. 3 shows that the peptide region (red) of A-FABP (green) used to produce antibodies served as an antigenic epitope binding to the 6H2 complementarity determining regions (CDRs) (CDRs of 6H2 heavy chain: yellow; 6H2 CDRs of the light chain: orange), Asn33 of the 6H2 heavy chain (6H2-H) was in contact with A-FABP. The structure also shows that 6H2 binds directly to the "lid" of the β-barrel (Ser14 to Ala37 of A-FABP), which is thought to control the entry of lipid into the binding pocket. Docking analysis shows that the activity of 6H2 may be related to A-FABP-lipid binding.

**Example 3**

This example is used to detect the activity of monoclonal antibody 6H2.

**1. Determination of binding affinity of 6H2 by Biacore**

The binding kinetics of 6H2 to human and mouse A-FABP antigens were analyzed by surface plasmon resonance (SPR) using a Biacore 8k (GE HealthCare, USA) instrument. The specific operations were performed as follows: The A-FABP antigen was covalently immobilized on the sensor chip using standard amine coupling chemistry. The twice diluted antibody was injected onto the channel at a flow rate of 30 μL/min for 3 minutes and allowed to dissociate for a further 10 to 15 minutes, followed by regeneration with the injection of 25 μL of 10 mM glycine-HCl (pH 2.5) at a flow rate of 30 μL/min. The data were processed and analyzed using Biacore 8K Evaluation Software version 1.0 (GE Healthcare Bio-Sciences). The results show that 6H2 exhibits moderate affinity for both human A-FABP and mouse A-FABP, with KD values of 2.57E-06 and 5.50E-07, respectively.

## 2. Specificity of 6H2 for different FABPs

The mouse or human A-FABP, E-FABP, and H-FABP recombinant proteins (1 $\mu$g/mL) were coated on 96-well plates overnight for ELISA binding assay to evaluate the reactivity and specificity of monoclonal antibodies with various FABPs.

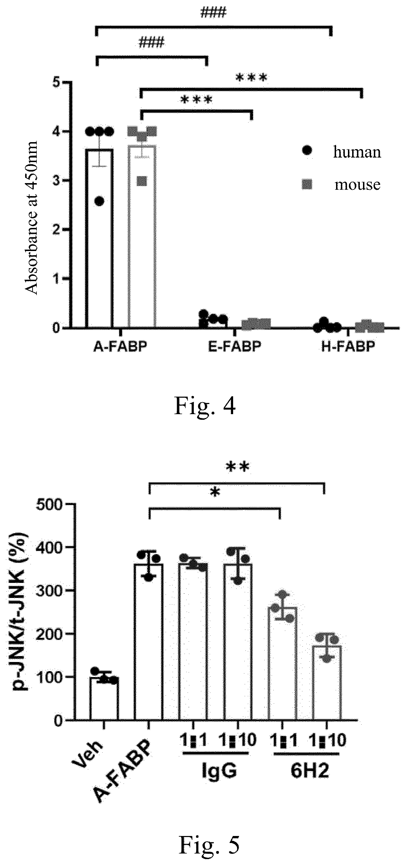

100 $\mu$L of 6H2 (1 $\mu$g/mL) was added to the wells and incubated for 1 h at room temperature. The plates were then washed three times with phosphate-buffered saline (PBS), followed by incubation with 0.2 $\mu$g/mL HRP-conjugated-mouse IgG H+L (Proteintech, SA00001-1, Japan) as the detection antibody at room temperature for 1 hour. After washing with PBS, TMB chromogen solution (Beyotime, P0209, China) was added for color development; 2M $H_2SO_4$ was added to the wells to stop the enzyme-induced color reactions; and the absorbance at 450 nm was measured with a microtiter plate reader. The results are shown in Fig. 4.

Fig. 4 shows that 6H2 is highly specific for human and mouse A-FABP, while showing no cross-reactivity with human and mouse epidermal-FABP (e-FABP) and heart-FABP (h-FABP).

## 3. Determination of neutralizing activity of the 6H2 monoclonal antibody

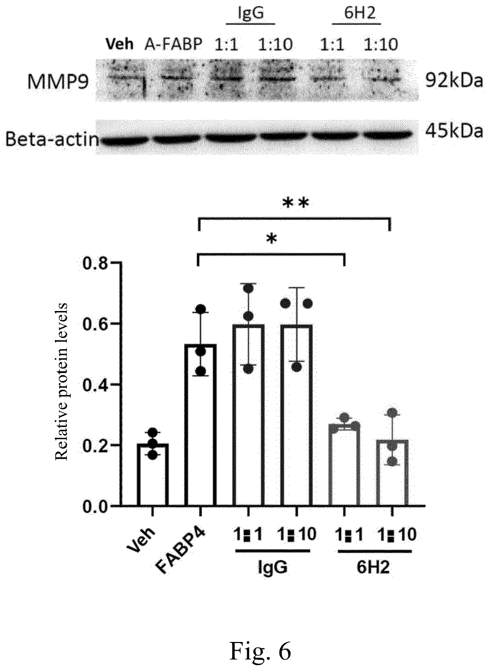

Previous studies have shown that A-FABP mediates inflammatory responses in various tissues by activating the JNK/c-Jun signaling pathway. To evaluate the neutralizing activity of 6H2 on the biological effects of A-FABP, ELISA and Western blot assays were performed to determine the p-JNK/JNK ratio and MMP9 production in mouse primary peritoneal macrophages after treatment with rA-FABP or mouse IgG as a negative control pre-incubated with 6H2 (1:1 and 1:10 ratios). The results are shown in Figs. 5 and 6.

Eight-week-old mice were intraperitoneally injected with 1 mL of 3.8% thioglycolate medium (BD, 211716, USA), and 3 days later, primary peritoneal macrophages were obtained by intraperitoneal lavage to evaluate the neutralizing activity of monoclonal antibody 6H2 in inhibiting A-FABP-mediated inflammation. Primary peritoneal macrophages were cultured in high glucose Dulbecco's Modified Eagle's Medium (DMEM) supplemented with 10% fetal bovine serum (FBS) and 100 U/mL penicillin and 100 $\mu$g/mL streptomycin at 37°C in 5% $CO_2$ incubator. The medium was replaced 6 hours after seeding in 96-well plates.

To determine the efficiency of 6H2 in inhibiting A-FABP-mediated activation of the JNK signaling pathway, 1 $\mu$g/mL mouse rA-FABP was pre-incubated with 6H2 (rA-FABP: 6H2 molar ratio of 1:1 or 1:10) or mouse non-immunoglobulin G (rA-FABP:IgG molar ratio of 1:1 or 1:10) in DMEM for 30 min at 37°C. Subsequently, primary peritoneal macrophages were treated with the above mixture for an additional 30 min at 37°C. The activation of JNK signaling in treated primary peritoneal macrophages was further analyzed by ELISA (n=3).

The p-JNK/JNK ratio in mouse primary peritoneal macrophages was detected using Phospho-JNK (T183/Y185) + Total In-Cell ELISA Kit (Abeam, ab207477, UK), and all solutions were prepared according to the manufacturer's manual. After treatment of mouse peritoneal macrophages as described in Test 3, the culture medium was removed and the cells were fixed with 4% formaldehyde. Cells were then treated with antibody-blocking buffer and incubated with primary and secondary antibodies. The plate was then developed with TMB chromogen solution (Beyotime Biotechnology, P0209, China). 2 M $H_2SO_4$ was added to each well to stop the enzyme-induced color reaction and the absorbance at 450 nm was measured with a microplate reader.

Figs. 5 and 6 show that treatment with rA-FABP (1 ug/mL) alone can significantly induce the p-JNK/JNK ratio, which is significantly inhibited when recombinant A-FABP was pre-incubated with 6H2 at a ratio of 1:1, and the p-JNK/JNK ratio was further down-regulated at the ratio of 1:10; similarly, the expression of MMP-9 induced by A-FABP was also significantly inhibited by 6H2 treatment at the ratio of 1:1 and 1: 10. However, pre-incubated mouse IgG did not show any effect. These data indicate that 6H2 exhibits strong neutralizing activity against A-FABP.

## 4. Determination of the half-life of 6H2 monoclonal antibody

To determine the half-life of monoclonal antibody 6H2, 6H2 was first biotin-labeled with Sulfo-NHS-Biotin (Thermo Scientific, A39256, USA). Unbound biotin was eliminated by desalting on a ZebaSpin desalting spin column (ThermoFisher, 89882, USA). 1.8 mg/kg of biotin-labeled 6H2 was intravenously injected into 8-week-old C57BL/6N mice, and blood samples were collected 1 hour and 1 to 15 days after injection. Western blot analysis was performed using HRP-conjugated streptomycin (Thermo Scientific, TH273544, USA) to detect the 6H2 heavy chain (6H2-H) and light chain (6H2-L). The serum biotin-labeled 6H2-H or 6H2-L content at different time points was represented as a percentage. The serum biotin-labeled 6H2-H or 6H2-L content at 1 hour after injection was defined as 100%, and the blank serum was defined as 0%. The half-life of 6H2 refers to the time to the biotin-labeled 6H2-H or 6H2-L content in the serum reaching 50%.

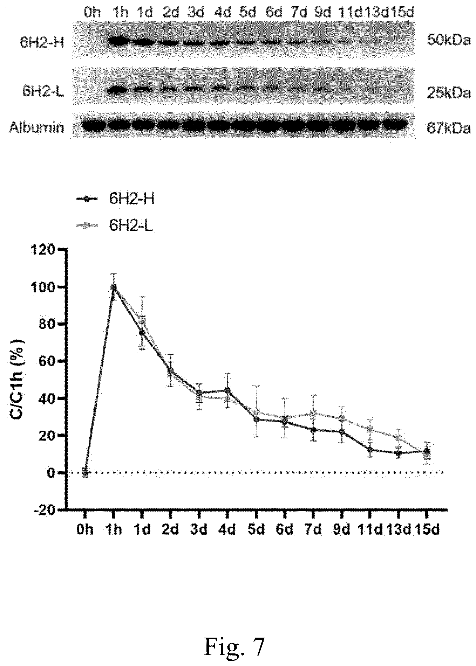

C57BL6/N mice were intravenously injected with biotin-labeled 6H2 or mouse IgG at a concentration of 1.8 mg/kg. Blood samples from mice were collected at different time points after injection of biotin-labeled 6H2 or mouse IgG. Western blot analysis showed that the half-life of 6H2 mAb was 2 to 3 days and could be maintained in serum for about 2 weeks. The results are shown in Fig. 7.

**Example 4**

This example is used to characterize the therapeutic effect of monoclonal antibody 6H2 on cerebral ischemia.

**1. 6H2 treatment alleviates brain injury caused by MCAO**

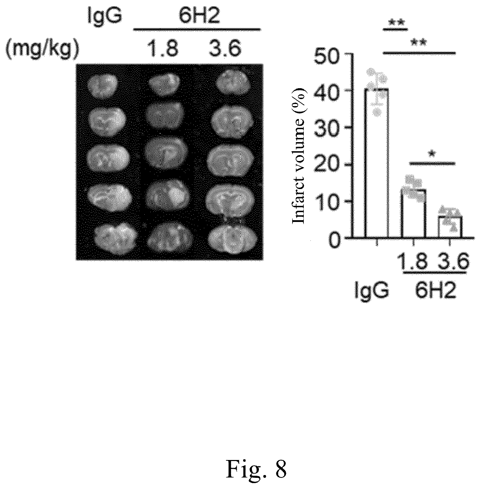

To evaluate the therapeutic potential of monoclonal antibody 6H2 for cerebral ischemia, C57BL/6N mice were subjected to MCAO surgery for 1 hour, followed by intravenous injection of 6H2 or mouse IgG, and reperfusion for 23 hours or 7 days. The dose of 6H2 was calculated based on the serum levels of A-FABP induced by MCAO. The TTC staining results 24 hours after MCAO occurred show that treatment with 1.8 mg/kg 6H2 could significantly reduce the infarct volume caused by ischemia, as shown in Fig. 8.

Further increasing the dose of 6H2 to 3.6 mg/kg did not show further reduction in infarct volume. Therefore, 1.8 mg/kg 6H2 was used in the following studies.

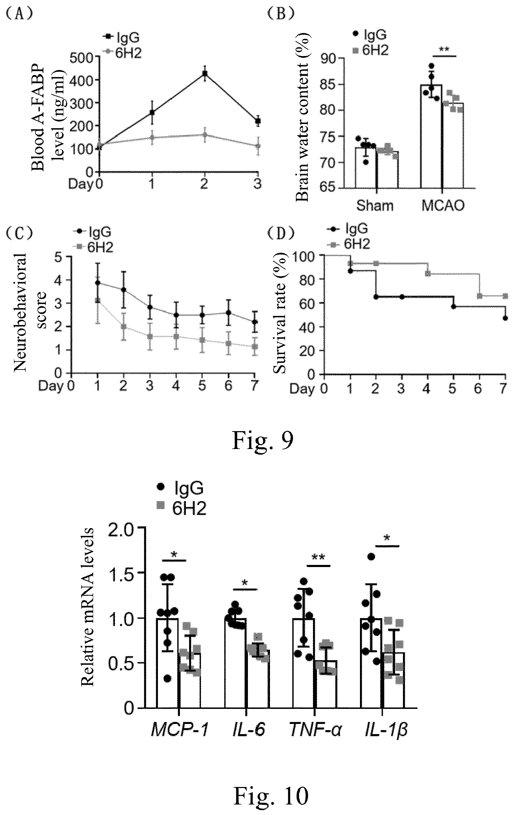

ELISA results show that treatment with 6H2 significantly reduced the ischemic stroke-induced increase in serum A-FABP compared with mice treated with mouse IgG, as shown in Fig. 9, Panel A. This data suggests that 6H2 effectively binds to circulating A-FABP, resulting in a decrease in free A-FABP detected by ELISA. Consistently, 6H2-treated mice exhibited a significant decrease in ischemia-induced brain edema compared with mouse IgG-treated mice, as shown in Fig. 9, Panel B. Long-term neurological deficits within 7 days after MCAO were also significantly improved, correlating with the increased survival rate of 6H2-treated mice. The results are shown in Fig. 9, Panels C and D. In addition, in 6H2-treated mice, the expression of pro-inflammatory cytokines in the brain caused by MCAO was significantly alleviated, as shown in Fig. 10. The above data are represented as mean $\pm$ SD, *P<0.05, **P<0.01.

The above data suggest that treatment with 6H2 neutralizes MCAO-induced circulating A-FABP, leading to a reduction in pro-inflammatory cytokine secretion, thereby attenuating brain injury and adverse functional outcomes. These data validate the broad potential applicability of neutralizing A-FABP to combat ischemic brain injury.

**2. 6H2 treatment alleviates blood-brain barrier disruption caused by A-FABP during MCAO surgery**

Previous studies found that the A-FA13P-JNK-MMP-9 signaling pathway exacerbates cerebral ischemic injury by promoting MCAO-induced blood-brain barrier disruption (BBB) leakage. Therefore, the role of 6H2 in rescuing BBB integrity was evaluated.

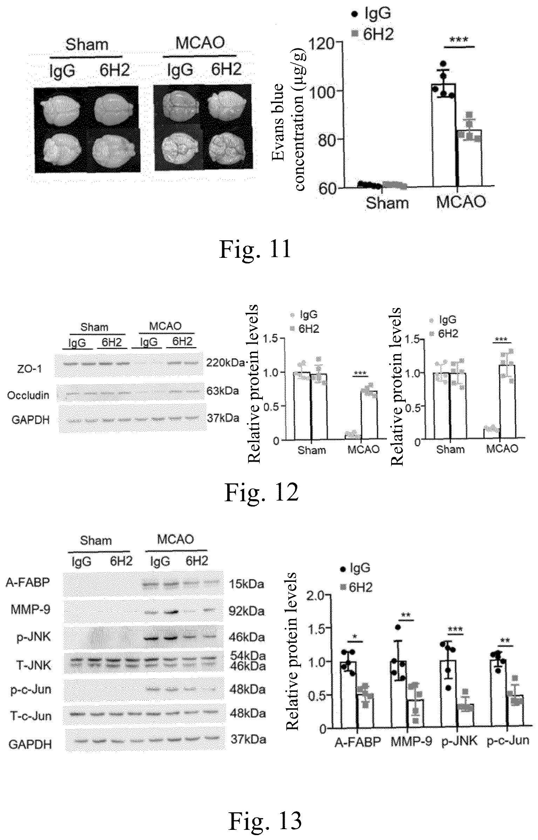

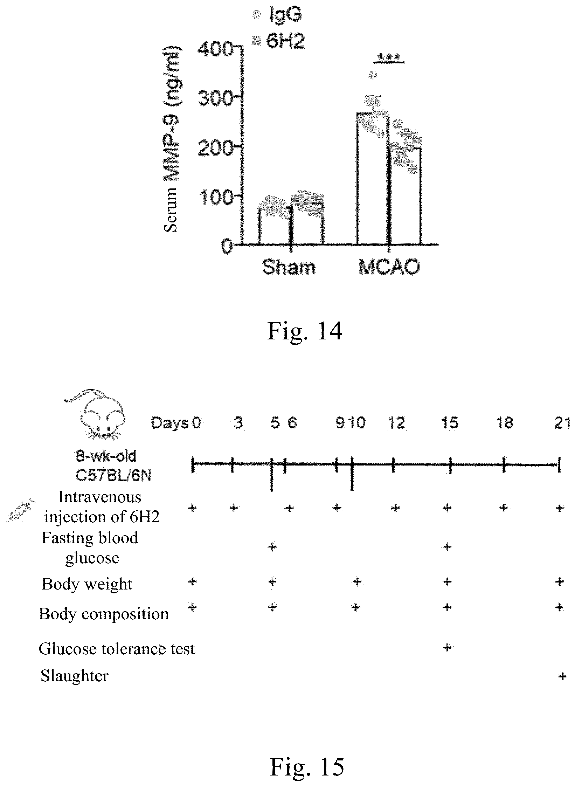

6H2 or mouse IgG was injected intravenously into mice 1 hour after MCAO surgery. Compared with the mouse IgG-treated control, ischemia-induced BBB leakage was significantly reduced in 6H2-treated mice, manifested by reduced permeability to Evans blue dye, as shown in Fig. 11. Ischemia-induced degradation of tight junction (TJ) proteins Occludin and ZO-1 was also significantly alleviated by 6H2, indicating that 6H2 has a protective effect on BBB leakage, as shown in Fig. 12. MCAO-induced activation of the JNK/c-Jun signaling pathway as well as MMP-9 levels in the brain and circulation during ischemic stroke were also significantly inhibited by 6H2, as shown in Figs. 13 and 14.

The above data are represented as mean $\pm$ SD, *p<0.05, **p<0.01, ***p<0.001.

**3. Long-term treatment with 6H2 does not affect the systemic metabolism of healthy mice**

To explore whether 6H2 treatment resulted in side effects, mice were intravenously injected with 6H2 (1.8 mg/kg) or mouse IgG (1.8 mg/kg) every 3 days for 21 days, and basic metabolic parameters were monitored, as shown in Fig. 15.

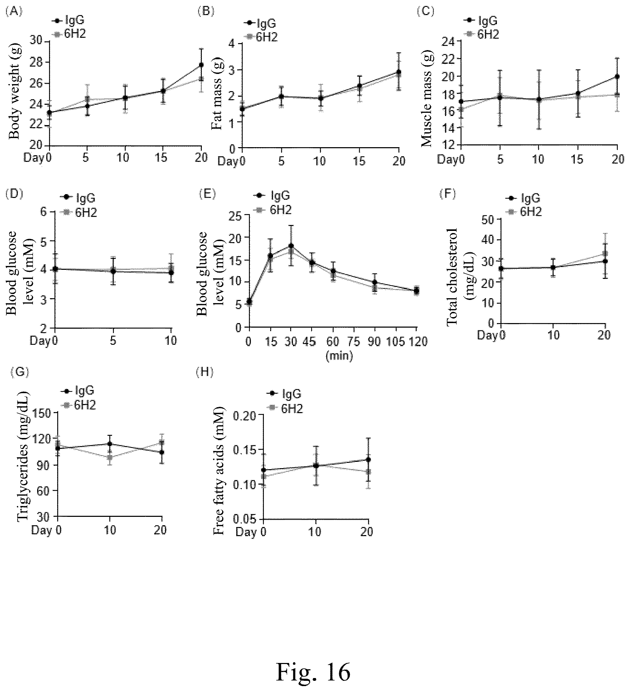

There were no significant changes in the body weight (Fig. 16, Panel A), fat (Fig. 16, Panel B), and muscle (Fig. 16, Panel C) as well as fasting blood glucose (Fig. 16, Panel D) of 6H2-treated mice, compared with mouse IgG-treated mice.

Glucose metabolism was verified using the glucose tolerance test (GTT). Compared with mouse IgG treatment, 6H2 treatment did not alter glucose tolerance, as demonstrated in similar glucose treatment curves (Fig. 16, Panel E). Since A-FABP is a lipid chaperone associated with lipid homeostasis, levels of total cholesterol (TC), triglyceride (TG), and free fatty acid (FFA) in serum were evaluated accordingly (Fig. 16, Panels F-H). No significant differences in the above parameters were observed between mouse IgG- and 6H2-treated groups. The above data demonstrate that 6H2 is a safe neutralizing monoclonal antibody for the treatment of ischemia-induced brain injury in mice.

The above examples showed that a single intravenous injection of 6H2 (1.8 mg/kg) 1 hour after middle cerebral artery occlusion (MCAO) surgery significantly alleviated the consequences of ischemic stroke, including reduced infarct size, brain edema, BBB leakage, inflammation, and improved neurological function and survival rate (1 day) in mice. Treatment with 6H2 every 3 days after ischemic stroke also improved long-term (7-day) survival rate in mice. Intravenous injection of 6H2 in healthy C57BL/6N mice for 21 days did not change any physiological parameters, including body composition, glucose levels, and lipid profile, compared with mice injected with IgG, indicating the safety of 6H2. These data demonstrate that 6H2 is a monoclonal antibody capable of being used in the treatment of ischemic stroke with high safety and efficacy.

The above examples are merely preferred embodiments of the present invention and do not limit the present invention in any way. Without departing from the scope of the technical solution of the present invention, any form of equivalent substitution or modification and other changes made by any person skilled in the art to the technical solution and technical content disclosed in the present invention does not depart from the technical solution of the present invention, and still falls within the protection scope of the present invention.

**References**

[1] M. Zhou, Y. Bao, H. Li, Y. Pan, L. Shu, Z. Xia, D. Wu, K.S. Lam, P.M. Vanhoutte, A. Xu, W. Jia, R.L. Hoo, Deficiency of adipocyte fatty-acid-binding protein alleviates myocardial ischaemia/reperfusion injury and diabetes-induced cardiac dysfunction, Clin Sci (Lond) 129(7) (2015) 547-59.
[2] Liao B, Geng L, Zhang F, Shu L, Wei L, Yeung PKK, et al. Adipocyte fatty acid-binding protein exacerbates cerebral ischaemia injury by disrupting the blood-brain barrier. Eur Heart J. 2020; 41(33):3169-80.
[3] Burak MF, Inouye KE, White A, Lee A, Tuncman G, Calay ES, et al. Development of a therapeutic monoclonal antibody that targets secreted fatty acid-binding protein aP2 to treat type 2 diabetes. Sci Transl Med. 2015; 7(319):319ra205.
[4] Cao H, Sekiya M, Ertunc ME, Burak MF, Mayers JR, White A, et al. Adipocyte lipid chaperone AP2 is a secreted adipokine regulating hepatic glucose production. Cell Metab. 2013; 17(5):768-78.

**Claims**

**1.** An A-FABP neutralizing monoclonal antibody or antigen-binding fragment thereof, comprising:

a complementarity determining region 1 of a heavy chain variable region, the amino acid sequence of which is set forth in SEQ ID NO. 1; a complementarity determining region 2 of the heavy chain variable region, the amino acid sequence of which is set forth in SEQ ID NO. 2; and a complementarity determining region 3 of the heavy chain variable region, the amino acid sequence of which is set forth in SEQ ID NO. 3, and/or
a complementarity determining region 1 of a light chain variable region, the amino acid sequence of which is set forth in SEQ ID NO. 4; a complementarity determining region 2 of the light chain variable region, the amino acid sequence of which is set forth in SEQ ID NO. 5; and a complementarity determining region 3 of the light chain variable region, the amino acid sequence of which is set forth in SEQ ID NO. 6.

**2.** The monoclonal antibody or antigen-binding fragment thereof according to claim 1, wherein the amino acid sequence of the heavy chain variable region of the antibody is set forth in SEQ ID NO. 7, and the amino acid sequence of the light chain variable region is set forth in SEQ ID NO. 8.

**3.** The monoclonal antibody or antigen-binding fragment thereof according to claim 1 or 2, wherein the antigen-binding fragment is selected from one or more of Fab, Fab', (Fab')2, Fv, disulfide-linked Fv, scFv, diabody, and single-domain antibody.

**4.** The monoclonal antibody or antigen-binding fragment thereof according to any one of claims 1 to 3, wherein the monoclonal antibody is selected from one or more of a murine antibody, a chimeric antibody, a humanized antibody, a bispecific antibody, or a multispecific antibody.

**5.** The antibody or antigen-binding fragment thereof according to any one of claims 1 to 4, wherein the monoclonal antibody is a murine antibody;
preferably, the amino acid sequence of the heavy chain variable region of the murine antibody is set forth in SEQ ID NO. 7, and the amino acid sequence of the light chain variable region is set forth in SEQ ID NO. 8.

6. The antibody or antigen-binding fragment thereof according to any one of claims 1 to 4, wherein the monoclonal antibody is a humanized antibody;

preferably, the humanized antibody comprises human IgG1, IgG2, IgG3 or IgG4; more preferably, the humanized antibody comprises human IgG4;
preferably, the amino acid sequence of the heavy chain of the humanized antibody is set forth in SEQ ID NO. 9, and the amino acid sequence of the light chain is set forth in SEQ ID NO. 10.

7. A preparation method for the monoclonal antibody or antigen-binding fragment thereof according to any one of claims 1 to 6, comprising:

(1) immunizing a mouse with a human recombinant A-FABP peptide;
(2) preparing hybridoma cells by fusing spleen cells from the immunized mouse in step (1) with myeloma cells;
(3) screening hybridoma cells which produce the A-FABP neutralizing monoclonal antibody.

8. The preparation method according to claim 7, wherein the immunizing is performed after mixing the human recombinant A-FABP peptide in step (1) with an adjuvant;

preferably, the screening in step (3) comprises: culturing with a hypoxanthine-aminopterin-thymidine medium, followed by screening hybridoma cells which produce the A-FABP neutralizing monoclonal antibody by enzyme-linked immunosorbent assay;
preferably, the preparation method further comprises:
(4) preparing the A-FABP neutralizing monoclonal antibody from mouse ascites and purifying with protein G magnetic beads.

9. A pharmaceutical composition for preventing and/or treating elevated circulating A-FABP or a disease or symptom resulting therefrom, comprising the monoclonal antibody or antigen-binding fragment thereof according to any one of claims 1 to 6, or a monoclonal antibody or antigen-binding fragment thereof prepared according to the preparation method of claim 7 or 8.

10. The pharmaceutical composition according to claim 9, further comprising a pharmaceutically acceptable carrier, diluent and/or adjuvant;

preferably, the pharmaceutical composition further comprises one or more other pharmaceutical active ingredients for preventing and/or treating elevated circulating A-FABP or a disease or symptom resulting therefrom;
preferably, the elevated circulating A-FABP or the disease or symptom resulting therefrom is selected from one or more of ischemic stroke, obesity, liver cirrhosis, arteriosclerosis, and diabetes.

11. A monoclonal antibody or antigen-binding fragment thereof according to any one of claims 1 to 6, or a monoclonal antibody or antigen-binding fragment thereof prepared according to the preparation method of claim 7 or 8, for use in a method of preventing and/or treating elevated circulating A-FABP or a disease or symptom resulting therefrom.

12. The monoclonal antibody or antigen-binding fragment thereof according to claim 11, wherein the monoclonal antibody or antigen-binding fragment thereof further comprises a pharmaceutically acceptable carrier, diluent and/or adjuvant;

preferably, the monoclonal antibody or antigen-binding fragment thereof further comprises one or more other pharmaceutical active ingredients for preventing and/or treating elevated circulating A-FABP or a disease or symptom resulting therefrom;
preferably, the elevated circulating A-FABP is selected from one or more of ischemic stroke, obesity, liver cirrhosis, arteriosclerosis, and diabetes.

Fig. 1

Fig. 2

Peptide region of A-FABP used to
generate antibodies

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Europäisches
Patentamt

European
Patent Office

Office européen
des brevets

# EUROPEAN SEARCH REPORT

Application Number

EP 23 20 9441

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIAO BOYA ET AL: "Adipocyte fatty acid-binding protein exacerbates cerebral ischaemia injury by disrupting the blood-brain barrier", EUROPEAN HEART JOURNAL, vol. 41, no. 33, 29 April 2020 (2020-04-29), pages 3169-3180, XP093146138, GB ISSN: 0195-668X, DOI: 10.1093/eurheartj/ehaa207 Retrieved from the Internet: URL:https://academic.oup.com/eurheartj/article-pdf/41/33/3169/46616990/eurheartj_41_33_3169.pdf> * page 3180, last paragraph; page 3169 - page 3178; figures 3,4 * | 1-12 | INV. C07K16/18 A61P25/00 A61K39/00 |
| X | MIAO XIAOLIANG ET AL: "The mAb against adipocyte fatty acid-binding protein 2E4 attenuates the inflammation in the mouse model of high-fat diet-induced obesity via toll-like receptor 4 pathway", MOLECULAR AND CELLULAR ENDOCRINOLOGY, vol. 403, 14 January 2015 (2015-01-14), pages 1-9, XP029197960, ISSN: 0303-7207, DOI: 10.1016/J.MCE.2014.12.017 * page 8, last paragraph bridging to page 9, first paragraph; page 7, column 1, paragraph 3; abstract; item 2.3; item 3.2; item 3.7; item 4 "Discussion"; figures 2-7 * | 1-12 | |

TECHNICAL FIELDS SEARCHED (IPC)

C07K
A61P
A61K

-/--

The present search report has been drawn up for all claims

| Place of search | Date of completion of the search | Examiner |
|---|---|---|
| Munich | 15 April 2024 | Klee, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches
Patentamt

European
Patent Office

Office européen
des brevets

# EUROPEAN SEARCH REPORT

Application Number

EP 23 20 9441

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 103 665 139 A (UNIV CHINA PHARMA) 26 March 2014 (2014-03-26) * 1st paragraph "Summary of the invention"; Embodiments 5-7"; claims 1-9 * | 1-12 | |
| A | CN 114 081 882 A (SHENZHEN INST OF ADV TECH CAS) 25 February 2022 (2022-02-25) * Paragraph 1 on "Background technique"; table 2 * | 1-12 | |
| X | WO 2005/029088 A2 (UNIV GENEVE; HOCHSTRASSER DENIS FRANCOIS [CH] ET AL.) 31 March 2005 (2005-03-31) * claims 16, 21; table 3; sequence 1 * | 1-12 | |
| X | WO 2016/176656 A2 (HARVARD COLLEGE [US]; UCB BIOPHARMA SPRL [BE]) 3 November 2016 (2016-11-03) * claims 1-152 * | 1-12 | TECHNICAL FIELDS SEARCHED (IPC) |

The present search report has been drawn up for all claims

| Place of search | Date of completion of the search | Examiner |
|---|---|---|
| Munich | 15 April 2024 | Klee, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 20 9441

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2024

| Patent document cited in search report | | Publication date | Patent family member(s) | | Publication date |
|---|---|---|---|---|---|
| CN 103665139 | A | 26-03-2014 | NONE | | |
| CN 114081882 | A | 25-02-2022 | CN 114081882 | A | 25-02-2022 |
| | | | WO 2023082397 | A1 | 19-05-2023 |
| WO 2005029088 | A2 | 31-03-2005 | AU 2004274710 | A1 | 31-03-2005 |
| | | | CA 2532130 | A1 | 31-03-2005 |
| | | | DK 2341350 | T3 | 05-02-2018 |
| | | | DK 2357477 | T3 | 05-02-2018 |
| | | | EP 1664795 | A2 | 07-06-2006 |
| | | | EP 2341350 | A1 | 06-07-2011 |
| | | | EP 2357477 | A1 | 17-08-2011 |
| | | | JP 5019878 | B2 | 05-09-2012 |
| | | | JP 2007506086 | A | 15-03-2007 |
| | | | US 2007042425 | A1 | 22-02-2007 |
| | | | WO 2005029088 | A2 | 31-03-2005 |
| WO 2016176656 | A2 | 03-11-2016 | AU 2016254215 | A1 | 26-10-2017 |
| | | | BR 112017023158 | A2 | 24-07-2018 |
| | | | CA 2982427 | A1 | 03-11-2016 |
| | | | CL 2017002737 | A1 | 25-05-2018 |
| | | | CN 107667116 | A | 06-02-2018 |
| | | | CO 2017012385 | A2 | 30-04-2018 |
| | | | EA 201792388 | A1 | 29-06-2018 |
| | | | EP 3288584 | A2 | 07-03-2018 |
| | | | JP 2018515082 | A | 14-06-2018 |
| | | | KR 20170138570 | A | 15-12-2017 |
| | | | SG 11201708323S | A | 29-11-2017 |
| | | | TW 201702271 | A | 16-01-2017 |
| | | | US 2016319003 | A1 | 03-11-2016 |
| | | | US 2019161536 | A1 | 30-05-2019 |
| | | | US 2022089705 | A1 | 24-03-2022 |
| | | | UY 36663 | A | 29-07-2016 |
| | | | WO 2016176656 | A2 | 03-11-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M. ZHOU ; Y. BAO ; H. LI ; Y. PAN ; L. SHU ; Z. XIA ; D. WU ; K.S. LAM ; P.M. VANHOUTTE ; A. XU.** Deficiency of adipocyte fatty-acid-binding protein alleviates myocardial ischaemia/reperfusion injury and diabetes-induced cardiac dysfunction. *Clin Sci (Lond),* 2015, vol. 129 (7), 547-59
- **LIAO B ; GENG L ; ZHANG F ; SHU L ; WEI L ; YEUNG PKK et al.** Adipocyte fatty acid-binding protein exacerbates cerebral ischaemia injury by disrupting the blood-brain barrier. *Eur Heart J.,* 2020, vol. 41 (33), 3169-80

- **BURAK MF ; INOUYE KE ; WHITE A ; LEE A ; TUNCMAN G ; CALAY ES et al.** Development of a therapeutic monoclonal antibody that targets secreted fatty acid-binding protein aP2 to treat type 2 diabetes. *Sci Transl Med.,* 2015, vol. 7 (319), 319-205
- **CAO H ; SEKIYA M ; ERTUNC ME ; BURAK MF ; MAYERS JR ; WHITE A et al.** Adipocyte lipid chaperone AP2 is a secreted adipokine regulating hepatic glucose production. *Cell Metab.,* 2013, vol. 17 (5), 768-78